# EUROPEAN PATENT APPLICATION

(11) **EP 3 106 457 A1**
(43) Date of publication of application: **21.12.2016**
(21) Application number: 15172082.8
(22) Date of filing: 15.06.2015
(51) Int. Cl.: C07C 315/04, C07F 5/02

(54) **A NOVEL SYNTHETIC PATHWAY TOWARDS APREMILAST**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to a process for providing a chiral β-hydroxysulfone compound, an intermediate useful in the synthesis of the isoindoline-based compound apremilast.

## Description

### Field of the invention

The present invention relates to enantioselective processes for providing a β-hydroxysulfone compound, a useful intermediate in the preparation of sulfone group containing isoindoline-based compound apremilast.

### Background of the invention

The synthesis of sulfone compounds and in particular a β-hydroxysulfone compound may provide a useful intermediate for the synthesis of apremilast, an anti-inflammatory agent active for treatment of psoriatic arthritis. Apremilast, which is *N*-{2-[(1*S*)-1-(3-ethoxy-4-methoxyphenyl)-2-(methylsulfonyl)ethyl]-1,3-dioxo-2,3-dihydro-1*H*-isoindol-4-yl}acetamide, is an inhibitor of PDE4. Apremilast specifically inhibits PDE4 and spontaneous production of TNF-α from human rheumatoid synovial cells and has anti-inflammatory activity.

WO 2000/25777 A1 describes the synthesis of the racemate of apremilast and derivatives thereof. In WO 2010/030345 A2 a synthesis of apremilast is described which *inter alia* includes the formation of a benzonitrile derivative and furthermore a reduction step. These processes use reagents which are not satisfactory for industrial synthesis.

WO 2013/126360 A2 and WO 2013/126495 A2 describe processes to produce apremilast which include the preparation of enantiomerically pure chiral β-aminosulfone intermediates using expensive as well as toxic materials such as metal catalysts, ligands or chiral auxiliaries.

WO 2003/080049 A1 describes the optical resolution of a racemic β-aminosulfone intermediate using in particular *N*-acetyl-L-leucine to obtain apremilast.

The object of the present invention is to provide short, simple, cost-effective, environmentally friendly and industrially suitable processes for beneficially providing a β-hydroxysulfone compound useful for the preparation of a sulfone group containing isoindoline-based compound apremilast.

### Summary of the invention

The object is solved by the methods according to claims 1, 10 and 15, the compound according to claim 13 and the use according to claim 14, while preferred embodiments are set forth in dependent claims and will be further described below.

The present invention in particular provides various aspects, advantageous features and preferred embodiments as summarized in the following items, which respectively alone or in combination particularly contribute to solving the object of the invention and eventually provide additional advantages:
(1) A process for providing a compound of formula V, 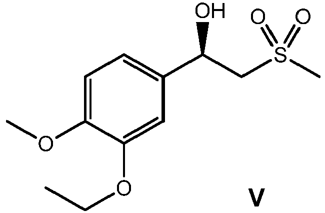 , comprising
   (a) providing a compound of formula III 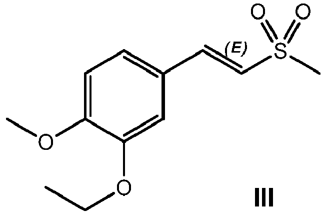 ,
   (b) reacting the compound of formula III with bis(pinacolato)diboron of formula 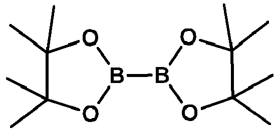 in the presence of a copper catalyst and a chiral phosphine ligand, to obtain a compound of formula IV 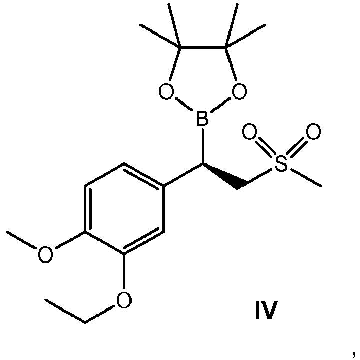 and
   (c) oxidizing the compound of formula IV to obtain the compound of formula V.
(2) The process according to item (1), wherein the compound of formula IV is not isolated.
(3) The process according to item (1), wherein the solvent used in steps (a) and (b) is an anhydrous aprotic or protic solvent.
(4) The process according to item (3), wherein the solvent is either anhydrous THF or anhydrous MeOH or combination thereof.

The term "anhydrous" solvent as used herein means a solvent, which contains less than 0.2 vol % of water, preferably less than 0.02 vol % of water.
(5) The process according to any of items (1) to (4), wherein the copper catalyst is selected from the group consisting of CuCl, Cu(CH₃CN)₄BF₄, Cu(OAc)₂, and basic CuCO₃.
(6) The process according to item (5), wherein the copper catalyst is CuCl.
(7) The process according to any of items (1) to (6), wherein the chiral phosphine ligand is selected from the group consisting of P-P ligands BINAP, PPhos, XylPPhos, ferrocenyl type P-P ligands of Josiphos and Walphos type, and phosphine-oxazoline ferrocenyl type ligand FerroPhox.
(8) The process according to any of items (1) to (7), wherein the chiral phosphine ligand is a P-P ligand selected from the group consisting of BINAP, PPhos, and XylPPhos.
(9) The process according to any of items (1) to (7), wherein the chiral phosphine ligand is ferrocenyl type P-P ligand of Josiphos or Walphos type.
(10) The process according to any of items (1) to (7), wherein the chiral phosphine ligand is a phosphine-oxazoline ferrocenyl type ligand FerroPhox.
(11) The process according to any of items (1) to (10), wherein the oxidizing agent is selected from the group consisting of H₂O₂/NaOH, potassium peroxymonosulfate, NaOCl, and NaBO₃ x ₄H₂O.
(12) The process according to item (11), wherein the oxidizing agent is NaBO₃ x 4H₂O.
(13) The process according to any of items (1) to (12), wherein the copper catalyst is CuCl, the chiral phosphine ligand is FerroPhox, and the oxidizing agent is NaBO₃ x 4H₂O.
(14) The process according to item (10) or (13), wherein the FerroPhox ligand has one of the following structures 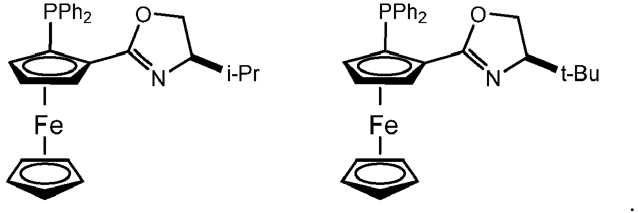
(15) The process according to items (10), (13) and (14), wherein the FerroPhox ligand is in (S,S) configuration.
(16) The process according to item (1), wherein the compound of formula III is obtained by providing the compound of formula I 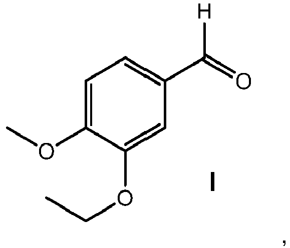 and converting the compound of formula I into the compound of formula III.
(17) The process according to item (16), wherein the compound of formula I is first converted to the compound of formula II 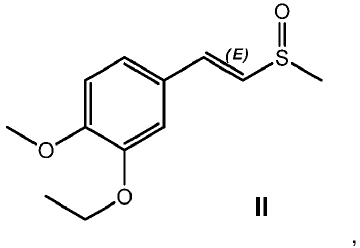 which is then further converted to the compound of formula III.
(18) A process for preparing apremilast 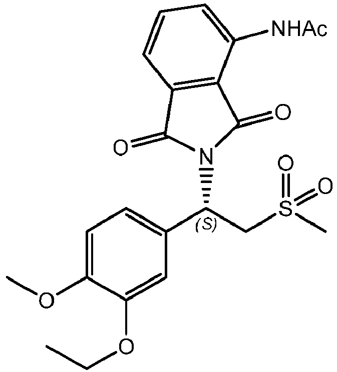 or a pharmaceutically acceptable salt or a solvate thereof, comprising:
   (aa) carrying out the process according to any one of the preceding items, and
   (bb) subsequently carrying out further synthetic step(s) to obtain apremilast.
(19) The process according to item (18), comprising
   (i)' converting the compound of formula V 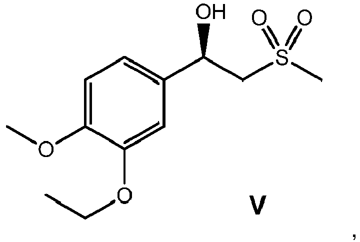 obtained by the process according to item (1), into a compound of formula VI 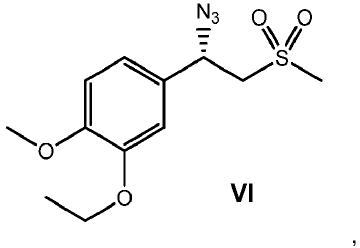
   (ii)' reducing the compound of formula VI to obtain a compound of formula VII 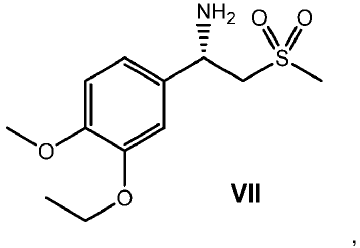 and
   (iii)' reacting the compound of formula VII with 3-acetamidophthalic anhydride of formula 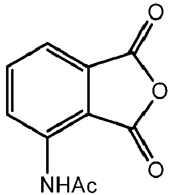 to obtain apremilast or a pharmaceutically acceptable salt or a solvate thereof.
(20) The process according to item (19), wherein in step (ii)' the reduction is carried out with triphenylphosphine in water.
(21) The process according to item (18), comprising
   reacting the compound of formula V 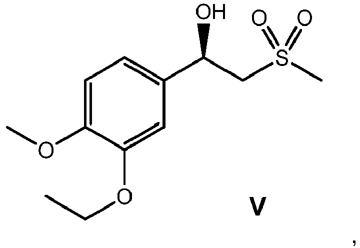 obtained by the process according to item (1), with 3-acetamidophthalimide of formula 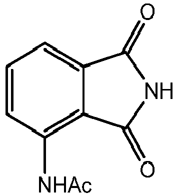
in the presence of a reagent selected from the group consisting of DEAD, DIAD and DCAD, in combination with Ph₃P and Bu₃P, to obtain apremilast or a pharmaceutically acceptable salt or a solvate thereof.
(22) A compound of formula IV 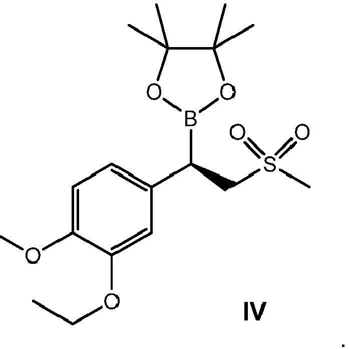
(23) Use of the compound as defined in item (22) in the preparation of apremilast or a pharmaceutically acceptable salt or a solvate thereof.
(24) A process for the preparation of a pharmaceutical composition comprising apremilast or a pharmaceutically acceptable salt or a solvate thereof, comprising the steps of:
   x) preparing apremilast or a pharmaceutically acceptable salt or a solvate thereof by carrying out a process according to item (18), and
   y) mixing said obtained apremilast or a pharmaceutically acceptable salt or a solvate thereof with a pharmaceutically acceptable carrier and/or excipient.

### Detailed description

In the following, the present invention is described in more detail while referring to preferred embodiments and examples, which are presented however for illustrative purposes and shall not be construed to limit the invention in any way.

A first aspect of the invention is a process for providing a compound of formula V

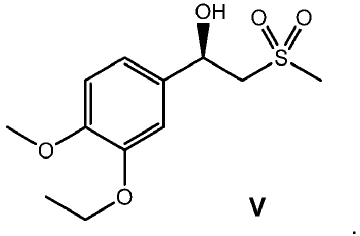

Said process is based on asymmetric β-borylation conjugate addition presented in Scheme 1.

The compound of formula III is reacted with bis(pinacolate)diboron (B₂pin₂) in the presence of a copper catalyst and a chiral ligand in a suitable solvent, for example, in an anhydrous protic or aprotic solvent, wherein the term anhydrous means a solvent, which contains less than 0.2 vol % of water. Next, the obtained compound of formula IV, which can optionally be isolated, is oxidized using a suitable oxidizing agent, for example, an oxidizing agent selected from the group consisting of H₂O₂/NaOH, potassium peroxymonosulfate, NaOCl, and NaBO₃ x 4H₂O, to yield the compound of formula V.

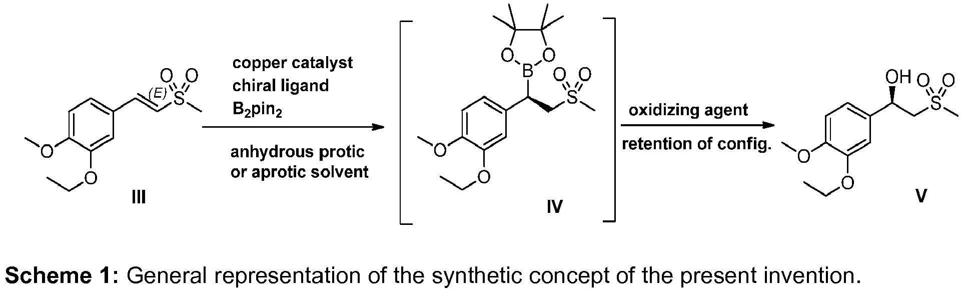

The copper catalyst used in the reaction is a catalyst commonly used for borylation reactions. For instance, Cu(I) or Cu(II) catalysts can be applied. Preferably, the copper catalyst can be selected from the group consisting of CuCl, Cu(CH₃CN)₄BF₄, Cu(OAc)₂, and basic CuCO₃. The chiral ligand used in the reaction is a chiral phosphine ligand, preferably selected from the group consisting of P-P ligands BINAP, PPhos, XylPPhos, ferrocenyl type P-P ligands of Josiphos and Walphos type, and phosphine-oxazoline ferrocenyl (FerroPhox) type ligands. The abbreviations of listed ligands represent chemical structures as defined in Scheme 2.

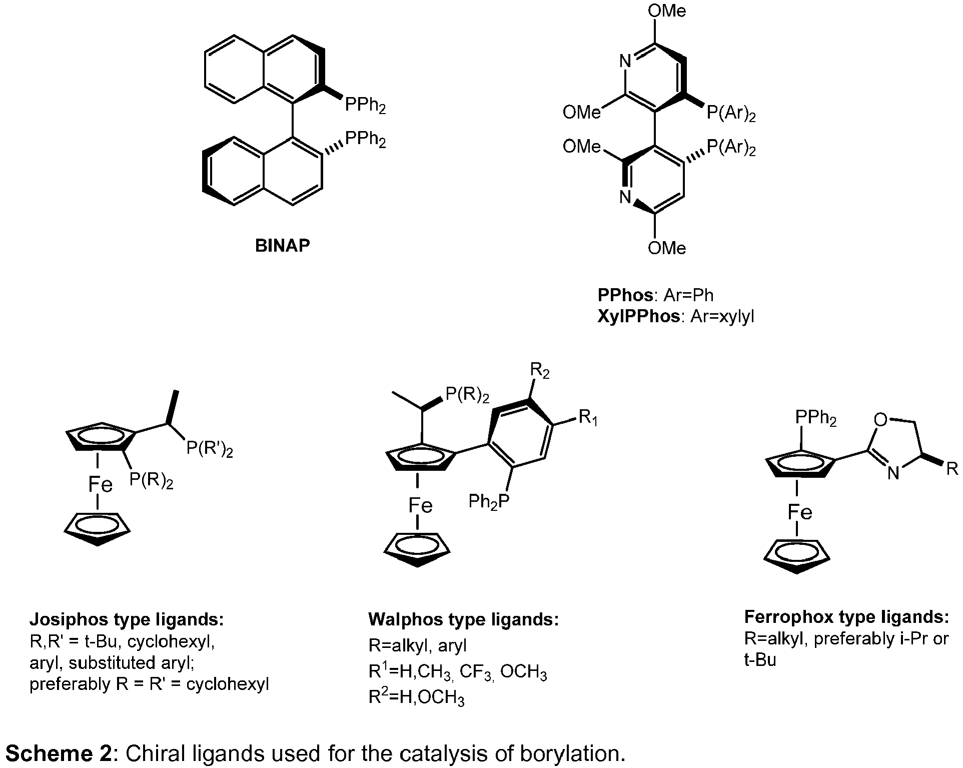

The term "alkyl" as used herein means straight, branched or cyclic hydrocarbons.

The term "aryl" as used herein means hydrocarbon aryls, preferably single or condensed six-membered rings, more preferably phenyl or naphthyl, in particular phenyl.

Particularly preferred chiral ligands are Ferrophox type ligands of the following structures:

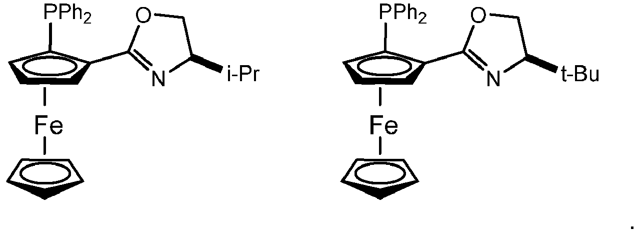

The anhydrous solvent, that is a solvent, which contains less than 0.2 vol % of water, preferably less than 0.02 vol % of water, can be selected from a group of protic solvents such as alcohols, preferably anhydrous methanol, or from aprotic solvents, preferably selected from ethers and aromatic hydrocarbons or a mixture thereof, most preferably the anhydrous aprotic solvent is tetrahydrofuran.

The purity of a chiral compound is measured by enantiomeric excess (ee), which is a degree to which one enantiomer is contained in a greater amount in a sample than the other enantiomer. Specifically, the enantiomeric excess is defined as the difference between the mole fractions of each enantiomer. Preferably, the one enantiomer provided by the process is obtained in an enantiomeric excess of at least 20%, more preferably at least 40%, even more preferably at least 60%, still more preferably at least 80%, yet more preferably at least 90%. Still more preferably the enantiomer obtained by the process is purified to at least 95% ee. It is particularly preferred that the enantiomer obtained by the process is pure. Pure enantiomer means that the one optical isomer of a compound is substantially free, preferably free, of the other enantiomer of the compound. Typically a pure enantiomer comprises greater than 98 wt.% of the one enantiomer and less than 2 wt.% of the other enantiomer, preferably greater than 99 wt.% of the one enantiomer and less than 1 wt.% of the other enantiomer.

The process according to the invention, which is preferably conducted at room temperature, provides for the compound of formula V with a high enantiomeric excess (ee), hence, there is no need for an additional optical resolution with a resolving agent. When, for example, the particularly preferred FerroPhox ligands are used in (*S,S*) configuration, then the compound of formula V is obtained in an ee of at least 95%.

The compound of formula III can be prepared from the compound of formula I directly or via the compound of formula II as presented in Scheme 3.

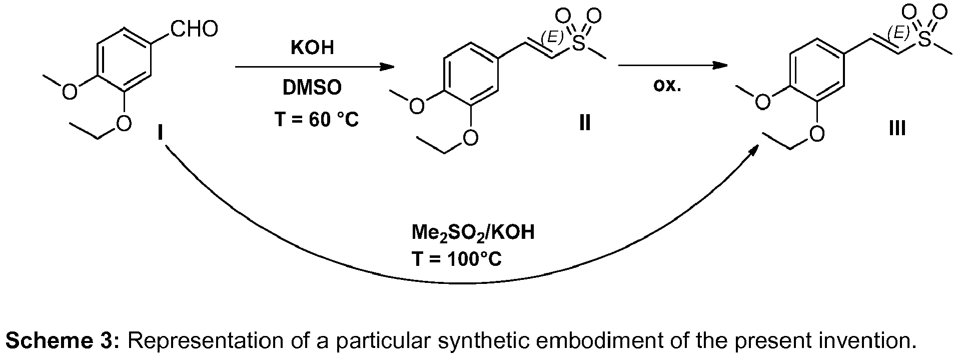

Preferably Me₂SO₂ and dimethyl sulfoxide (DMSO) are used in the condensation reaction with the benzaldehyde derivatives. Me₂SO₂ and DMSO can be further activated using a base, well known in the art, for example potassium *tert*-butoxide, KOH, Et₃N, Cs₂CO₃ or BuLi may be used.

Use of DMSO and Me₂SO₂ provides the respective methylsulfonyl and methylsulfinyl compounds. Specifically, using Me₂SO₂ the methylsulfonyl compound can be directly obtained. When DMSO is used the obtained methylsulfinyl compound is further converted to the methylsulfonyl compound by oxidation. For example, after the condensation formic acid and H₂O₂ can be added to the reaction mixture such that the methylsulfonyl compound is formed.

In another aspect of the present invention a process is provided for preparing apremilast or a pharmaceutically acceptable salt or solvate thereof, comprising carrying out the process according to the invention to obtain the compound of formula V, and subsequently carrying out further synthetic step(s) to obtain apremilast.

More specifically, apremilast can be prepared from the compound of formula V by following a process presented in Scheme 4.

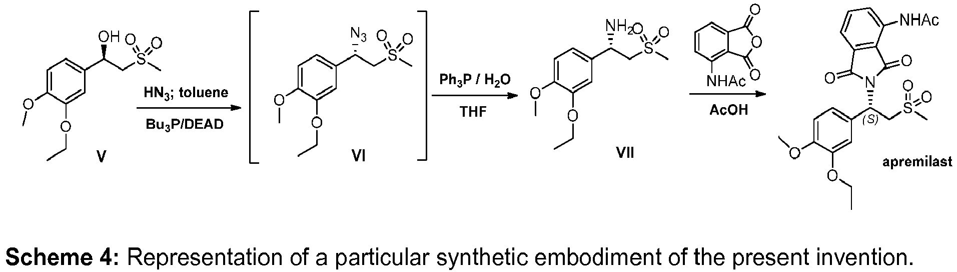

In the first step, the compound of formula V is contacted with a suitable solvent, for example, toluene and THF and then the mixture is flushed with N₂. This is followed by adding a solution of HN₃ in toluene and cooling the so obtained mixture. Once a temperature below -70°C is reached, tributylphosphine (Bu₃P) and diethylazodicarboxylate (DEAD) are added and the mixture is stirred at a temperature below -70°C for four hours. The mixture is then let to reach the room temperature, the solvents are evaporated and the obtained residue is dissolved in ethyl acetate and washed with brine and heptane to yield the compound of formula VI. Next, the obtained compound of formula VI is dissolved in THF and contacted with triphenylphosphine (Ph₃P) in water and stirred under N₂ atmosphere overnight. After solvent evaporation, ethyl acetate is added to a two-phase mixture of water and yellow oil and the mixture is washed with brine. Na₂SO₄ is used to dry the organic phase to yield a yellow solid residue. After another round of purification, finally white crystals of the compound of formula VII are obtained.

By following said process, the compound of formula VII with ee of around 80% can be obtained. To further improve the optical purity, N-acetyl-L-leucine may be utilized to give a stereochemically pure compound of formula VII with an ee of around 99%.

In the last step the obtained compound of formula VII is converted to apremilast by following processes known in the art, for example, referring to WO 00/25777 A1 and WO 03/080049 A1, apremilast can be obtained in a synthesis using 3-acetamidophthalimide and the chiral amino acid salt of the (S) enantiomer of the β-aminosulfone compound of formula VII, e.g. carried out in acetic acid.

Alternatively, apremilast can be prepared directly from the compound of formula V by following the Mitsunobu reaction as presented in Scheme 5.

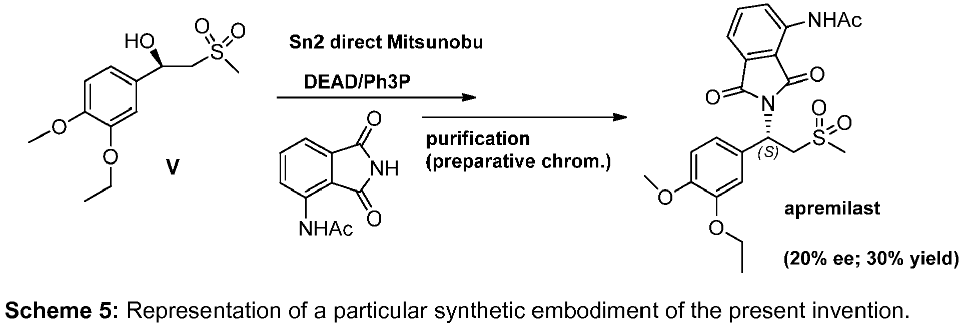

The compound of formula V is coupled with 3-acetamidophthalimide in the presence of an azodicarboxylate type reagent selected from the group consisting of DEAD (diethyl azodicarboxylate), DIAD (diisopropyl azodicarboxylate), and DCAD (di(4-chlorobenzyl) azodicarboxylate), in combination with Ph₃P and Bu₃P to yield crude apremilast. Using preparative chromatography as a purification method, pure apremilast can be obtained.

Further embodiment of the present invention resides in the provision of a valuable intermediate compound IV useful in the synthesis of apremilast or a pharmaceutically acceptable salt or a solvate thereof

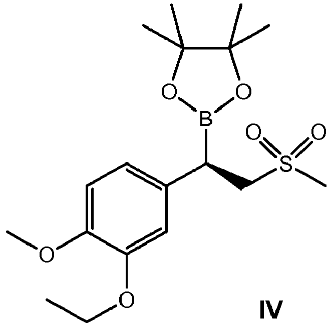

Another aspect of the invention is a process for preparing a pharmaceutical composition comprising apremilast or a pharmaceutically acceptable salt or solvate thereof, the process comprising the steps of carrying out the process of the invention to obtain apremilast or a pharmaceutically acceptable salt or solvate thereof, and mixing apremilast or a pharmaceutically acceptable salt or solvate thereof obtained according to the invention, optionally with another active pharmaceutical ingredient, with a pharmaceutically acceptable excipient, carrier and/or diluent.

In particular, apremilast or a pharmaceutically acceptable salt or solvate thereof is admixed with at least one suitable pharmaceutically acceptable excipient. Pharmaceutically acceptable excipients may be selected from the group consisting of diluents, carriers, binders, disintegrating agents, stabilizing agents, preservatives, lubricants, surfactants, fragrances, flavouring agents, sweeteners and other excipients known in the field of the pharmaceutical technology. For example, suitable excipients may be selected from the group consisting of lactose, microcrystalline cellulose, cellulose derivatives, such as hydroxypropylcellulose, polyacrylates, calcium carbonate, starch, colloidal silicone dioxide, sodium starch glycolate, croscarmellose sodium, talc, magnesium stearate, polyvinylpyrrolidone, polyethylene glycol and other excipients known in the field of the pharmaceutical technology.

Overall, in the present invention a simple and robust enantioselective synthesis of the β-hydroxy sulfone intermediate is provided. The obtained stereochemically pure β-hydroxy sulfone intermediate is used in a process to obtain apremilast using simple and robust chemical transformations. Simple and commercially available reagents and catalysts are used, and the intermediates may be formed directly without the need for additional protection/deprotection steps. Hazardous, toxic and expensive chemicals can favourably be avoided. In addition, there is no need for cumbersome preparatory equipment or techniques. Overall, the present invention thus provides cost-effective, environmentally friendly and industrially suitable synthetic processes.

### Examples

The following examples are merely illustrative of the present invention and they should not be considered as limiting the scope of the invention in any way. The examples and modifications or other equivalents thereof will become apparent to those versed in the art in the light of the present entire disclosure.

### Example 1: Synthesis of unsaturated sulfoxide (E)-2-ethoxy-1-methyl-4-(2-(methylsulfinyl)-vinyl)benzene (II) followed by oxidation to E)-2-ethoxy-1-methyl-4-(2-(methylsulfonyl)-vinyl)benzene (III)

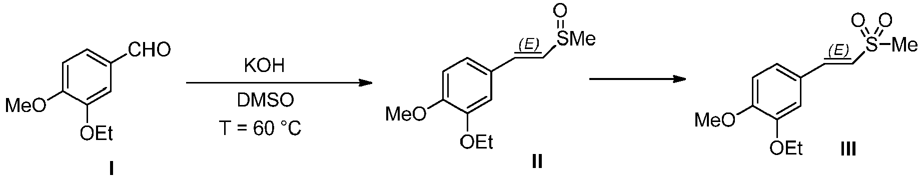

Into a 100 mL glass reactor equipped with a magnetic stir bar KOH was placed (1 equiv. according to starting material), suspended in anhydrous DMSO (30 mL) and then reaction mixture was stirred for 15 minutes at room temperature. Afterwards the starting material 3-ethoxy-4-methoxy-benzaldehyde (27 mmol, 4.87 g) was added in two portions and such reaction mixture was vigorously stirred at 60 °C for 3 hours. The reaction system was cooled down to room temperature, diluted with water and extracted with EtOAc (50 mL). Organic phases were washed with brine, dried over anhydrous Na₂SO₄ and solvent was evaporated under reduced pressure. The obtained crude product was dissolved in CH₂Cl₂ (22 mL) and formic acid (1.1 mL) was added. Afterwards the reaction system was cool down to 0 °C where 30% aqueous H₂O₂ was slowly added and reaction system was stirred at 27 °C overnight. The reaction system was diluted with water, neutralized with saturated solution of NaHCO₃ and then extracted with CH₂Cl₂. Organic phases were washed with brine, dried over anhydrous Na₂SO₄ and solvent was evaporated under reduced pressure where solid orange material was obtained as a crude product. Crude product was recrystallized from MTBE (4.93 g, 69% yield) and analysed using GC-MS (272 m/z) and ¹H NMR spectroscopy.
¹H NMR (500 MHz, CDCl₃) δ 7.50 (d, *J* = 15.4 Hz, 1H), 7.10 (dd, *J* = 8.4 Hz, *J* = 2.0 Hz, ArH), 7.00 (d, *J* = 2 Hz, ArH), 6.90 (d, *J* = 8.4 Hz, ArH), 6.80 (d, *J* = 15.4 Hz, 1H), 4.13 (q, *J* = 7.0 Hz, 2H), 3.90 (s, 3H), 3.05 (s, 3H), 1.50 (t, *J* = 7 Hz, 3H).

### Example 2: Direct Synthesis of (E)-2-ethoxy-1-methyl-4-(2-(methylsulfonyl)vinyl)benzene (III)

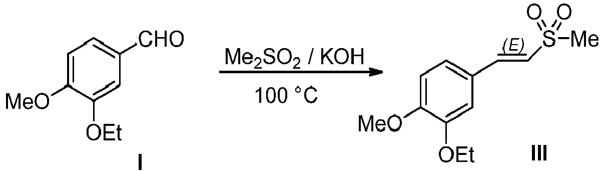

Into a 100 mL glass reactor equipped with magnetic stir bar were placed dimethylsulfone (15 equiv. according to starting material), KOH (1.05 equiv.) and were suspended in DMF (50 mL) Reaction mixture was stirred for 15 minutes at room temperature. Afterwards the starting material 3-ethoxy-4-methoxy-benzaldehyde (27 mmol, 4.87 g) was added in two portions and such reaction mixture was vigorously stirred at 100 °C for 2 hours. The reaction system was cool down to room temperature, diluted with saturated aqueous solution of NH₄Cl and extracted with EtOAc (2 x 60 mL). Organic phases were washed with brine, dried over anhydrous Na₂SO₄ and solvent was evaporated under reduced pressure. The obtained crude product was suspended in acetone/water and filtered off to afford yellowish solid material which was analysed and confirmed using NMR spectroscopy.
¹H NMR (500 MHz, CDCl₃) δ 7.50 (d, *J* = 15.4 Hz, 1H), 7.10 (dd, *J* = 8.4 Hz, *J* = 2.0 Hz, ArH), 7.00 (d, *J* = 2 Hz, ArH), 6.90 (d, *J* = 8.4 Hz, ArH), 6.80 (d, *J* = 15.4 Hz, 1H), 4.13 (q, *J* = 7.0 Hz, 2H), 3.90 (s, 3H), 3.05 (s, 3H), 1.50 (t, *J* = 7 Hz, 3H);
¹³C NMR (125 MHz, CDCl₃, ppm) δ 152.2, 148.6, 144.0, 124.7, 123.4, 111.2, 64.4, 55.9, 43.5, 14.7.

### Example 3: One-pot copper(I)-catalysed enantioselective synthesis of optically active 1-(3-ethoxy-4-methoxyphenyl)-2-(methylsulfonyl)ethanol (V) via β-borylation approach

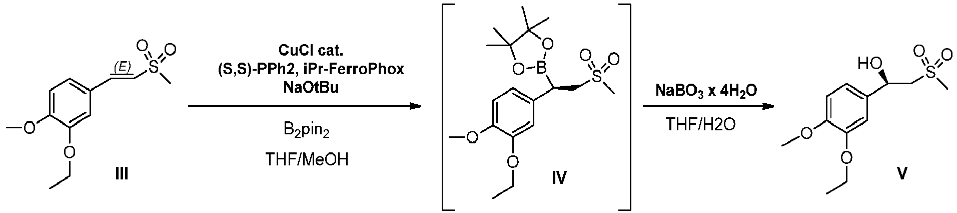

Into a 10 mL glass tube equipped with magnetic stir bar were placed Cu source (9 mol % of fresh CuCl), chiral P-N ligand (*S,S*)-PPh₂, ⁱPr-FerroPhox (6 mol %), base NaO^{t}Bu (9.4 mol %), starting material **(III)** (150 mg; 5.9 x 10⁻⁴ mol) and B₂pin₂ (168 mg, 6.6 x 10⁻⁴ mol) and test tube was sealed. The tube was efficiently purged with nitrogen. Meanwhile freshly degassed anhydrous THF (3 mL) and degassed anhydrous MeOH (50 µL) were added and reaction mixture was stirred for 4 hours at room temperature under constant flow of inert nitrogen atmosphere. Afterwards 277 mg (1,8 mmol) NaBO₃ x 4H₂O was weighed into a separate round bottom flask and 3 mL of newly degassed anhydrous MeOH was added. The flask was sealed with a rubber seal and liquid was purged with nitrogen while vigorously stirred. The reaction mixture from the tube was transferred to the flask with a syringe and stirred under nitrogen atmosphere overnight to obtain black solution. Organic solvents were removed *in vacuo* and organic residue was extracted with two portions (2x20 mL) of dicloromethane and washed with water. Combined organic phases were washed with brine, dried over MgSO₄ and organic solvent was removed under reduced pressure. The obtained crude solid material (80% of isolated yield) was analysed using chiral HPLC (96% ee was obtained) and confirmed with ¹H NMR.

### Example 4: One-pot copper(I)-catalysed enantioselective synthesis of optical active 1-(3-ethoxy-4-methoxyphenyl)-2-(methylsulfonyl)ethanol (V) via β-borylation approach

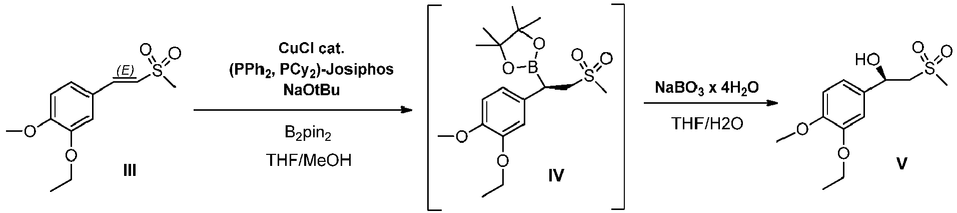

Into a 10 mL glass tube equipped with magnetic stir bar were placed Cu source (10 mol % of fresh (Cu(CH₃CN)₄)BF₄), chiral P-P ligand (PPh₂, PCy₂)-*Josiphos* (10 mol %), base NaO^{t}Bu (12 mol %), starting material **(III)** (150 mg; 5.9 x 10⁻⁴ mol) and B₂pin₂ (168 mg, 6.6 x 10⁻⁴ mol) and test tube was sealed. The tube was efficiently purged with nitrogen. Meanwhile freshly degassed anhydrous THF (3 mL) and degassed anhydrous MeOH (50 µL) were added and reaction mixture was stirred for 15 hours at room temperature under constant flow of inert nitrogen atmosphere. Afterwards 277 mg (1,8 mmol) NaBO₃ x 4H₂O was weighed into a separate round bottom flask and 3 mL of newly degassed anhydrous MeOH was added. The flask was sealed with a rubber seal and liquid was purged with nitrogen while vigorously stirred. The reaction mixture from the tube was transferred to the flask with a syringe and stirred under nitrogen atmosphere overnight to obtain black solution. Organic solvents were removed *in vacuo* and organic residue was extracted with two portions (2x20 mL) of dicloromethane and washed with water. Combined organic phases were washed with brine, dried over MgSO₄ and organic solvent was removed under reduced pressure. The obtained crude solid material (70% of isolated yield) was analysed using chiral HPLC (85% ee was obtained) and confirmed with ¹H NMR.

### Example 5: One-pot copper(I)-catalysed enantioselective synthesis of optical active 1-(3-ethoxy-4-methoxyphenyl)-2-(methylsulfonyl)ethanol (V) via β-borylation approach

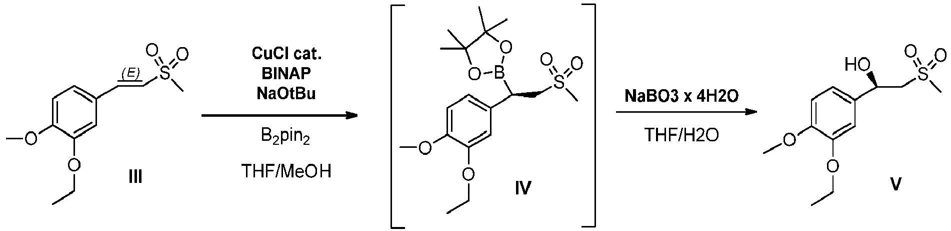

Into a 10 mL glass tube equipped with magnetic stir bar were placed Cu source (15 mol % of fresh CuCl), chiral P-P ligand BINAP (16 mol %), base NaO^{t}Bu (20 mol %), starting material **(III)** (150 mg; 5.9 x 10⁻⁴ mol) and B₂pin₂ (168 mg, 6.6 x 10⁻⁴ mol) and test tube was sealed. The tube was efficiently purged with nitrogen. Meanwhile freshly degassed anhydrous THF (3 mL) and degassed anhydrous MeOH (50 µL) were added and reaction mixture was stirred for 24 hours at room temperature under constant flow of inert nitrogen atmosphere. Afterwards 277 mg (1,8 mmol) NaBO₃ x 4H₂O was weighed into a separate round bottom flask and 3 mL of newly degassed anhydrous MeOH was added. The flask was sealed with a rubber seal and liquid was purged with nitrogen while vigorously stirred. The reaction mixture from the tube was transferred to the flask with a syringe and stirred under nitrogen atmosphere overnight to obtain black solution. Organic solvents were removed *in vacuo* and organic residue was extracted with two portions (2x20 mL) of dicloromethane and washed with water. Combined organic phases were washed with brine, dried over MgSO₄ and organic solvent was removed under reduced pressure. The obtained crude solid material was analysed using chiral HPLC (55% ee was obtained).

### Example 6: Preparation of compound VI

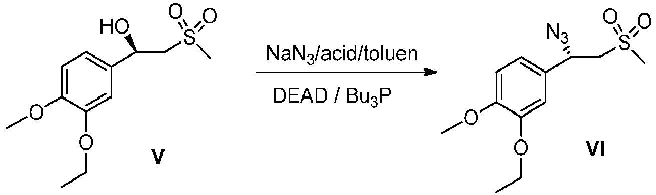

Optically active 1-(3-ethoxy-4-methoxyphenyl)-2-(methylsulfonyl)ethanol (**V**) (0,20 mg; 0,73 mmol) was weighed into a three-necked round-bottom flask, equipped with a thermometer. Toluene (56 mL) and fresh THF (4 mL) were added and the mixture was flushed with N₂ for 10 minutes. Freshly prepared solution of HN₃ in toluene (4,4 mL; 4,38 mmol) was added and the mixture was cooled in ethyl acetate - liquid N₂ bath. When the internal temperature reached less than -70° C, tributylphosphine (0,36 mL; 1,46 mmol) and then a solution of diethyl azodicarboxylate in toluene (0,77 mL; 1,68 mmol) were added and the mixture was stirred for additional 4 hours below -70 °C. After additional 18 hours the temperature reached room temperature. The solvents were evaporated under reduced pressure and the yellow oily residue was dissolved in 25 mL of ethyl acetate and washed three times with 25 mL of brine. The organic layer was dried over Na₂SO₄, filtered and the solvent was evaporated under reduced pressure to obtain yellow oil, which slowly solidified at room temperature. The solid residue was then triturated with 5 mL of heptane to obtain white crystals. The product (76% of isolated yield) was confirmed with ¹H NMR analysis and was used without additional purification in the next step using telescoping approach.
¹H NMR (500 MHz, CDCl₃) δ = 1,51 (t; *J* = 7,0 Hz; 3H; CH₂C*H*₃), 2,99 (s; 3H; SO₂CH₃), 3,17 (dd; *J* = 14,9 Hz, 3,7 Hz; 1 H; CHₐ), 3,45 (dd; *J* = 14,9 Hz, 9,9 Hz; 1 H; CH_{b}), 3,91 (s; 3H; OCH₃), 4,14 (q; *J* = 7,0 Hz; 2H; C*H*₂CH₃), 5,08 (dd; *J* = 9,9 Hz, 3,7 Hz; 1 H; CHN₃), 6,84 (s; 1 H; C₍₅₎H), 6,92 (s; 2H; C₍₂₎H, C₍₆₎H).

### Example 7: Synthesis of optically active 1-(3-ethoxy-4-methylphenyl)-2-(methylsulfonyl)-ethanamine (VII) using triphenylphoshine in water

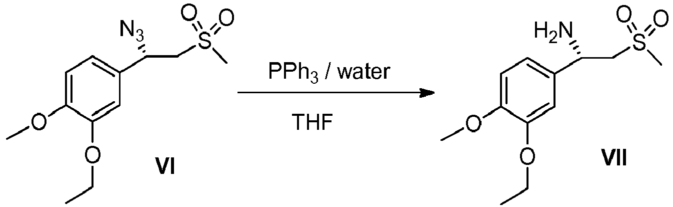

Triphenylphosphine (0,29 mg; 1,10 mmol) was weighed into a 50-mL round-bottom flask. Crude optical active compound **VI** (0,39 mg; 0,73 mmol) was dissolved in 10 mL of THF and added to the flask. Afterwards water (1 mL) was added and then the flask was sealed with a rubber septum and reaction mixture was stirred under N₂ atmosphere overnight. Afterwards solvent was evaporated under reduced pressure to obtain a two phase mixture of water and yellow oil. Ethyl acetate (20 mL) was added and washed twice with 20 mL of brine. The organic layer was dried over Na₂SO₄, filtered and evaporated under reduced pressure to obtain yellow solid residue. NMR analysis indicated the presence of iminophosphorane intermediate. Therefore the residue was dissolved in 10 mL of THF and 1 mL of water, transferred the mixture to a 50-mL round bottom flask equipped with a reflux condenser and stirred under reflux overnight. The solvent was evaporated under reduced pressure, 20 mL of ethyl acetate was added to the residue and washed three times with 10 mL of 1 M HCl. Combined aqueous layers were neutralized with K₃PO₄ until the pH was approximately 9 and then washed three times with 15 mL of ethyl acetate. The last three organic layers were combined, dried over Na₂SO₄, filtered off and evaporated under reduced pressure to obtain white crystals with 55% of isolated yield. Material was than dissolved in 1 mL of methanol and was heated to 80 °C over a period of 30 min. When the temperature reached between 35 and 40 °C, *N*-acetyl-L-leucine (0.6 equiv.) was added and the vial was sealed with a metal cap with septum. The mixture was stirred at 80 °C for 1 h and then cooled down to 20 °C in 2-3 hours. The white suspension was filtered through a Hirsch funnel (pore size No. 4) to obtain white solid material which was neutralized using NaOH in MTBE. Chiral HPLC analysis confirmed (*S*)-1-(3-ethoxy-4-methylphenyl)-2-(methylsulfonyl)ethanamine **(VII)** with 99% optical purity. Compound **VII** was confirmed also with ¹H NMR spectroscopy.
¹H NMR (500 MHz, CDCl₃) δ = 1,48 (t; *J* = 7,0 Hz; 3H; CH₂C*H*₃), 2,92 (s; 3H; SO₂CH₃), 3,23 (dd; *J =* 14,1 Hz, 3,3 Hz; 1 H; CHₐ), 3,34 (dd; *J =* 14,1 Hz, 9,5 Hz; 1 H; CH_{b}), 3,87 (s; 3H; OCH₃), 4,11 (q; *J* = 7,0 Hz; 2H; C*H*₂CH₃), 4.61 (dd; *J* = 9,5 Hz, 3,3 Hz; 1 H; C*H*NH₂), 6,85 (d; *J* = 8,2 Hz; 1 H; C₍₅₎H), 6,91 (dd; *J* = 8,2 Hz, 2,0 Hz; 1 H; C₍₆₎H), 6,93 (d; *J* = 2,0 Hz; 1 H; C₍₂₎H)

### Example 8: Preparation of (S)-1-(3-ethoxy-4-methoxyphenyl)-2-methylsulfonylethyl)-4-amino-isoindoline-1,3-dione (apremilast) from the compound of formula VII

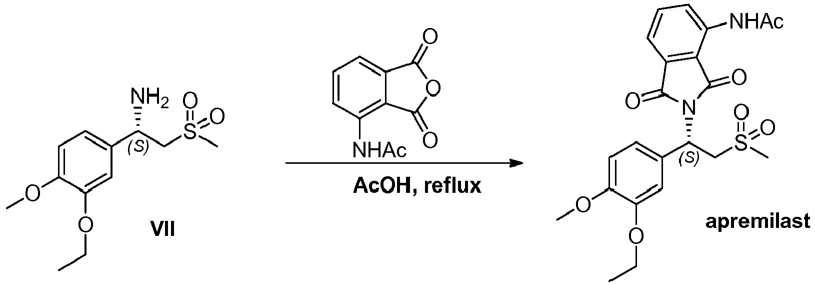

Into a reaction vessel equipped with magnetic stirrer and condenser was placed (S)-1-(3-ethoxy-4-methoxyphenyl)-2-(methylsulfonyl)ethanamine (2.24 mmol, 1 g) and was dissolved in 10 mL of glacial acetic acid. Afterwards 3-acetamidophthalic anhydride was added (2.35 mmol, 0.48 g) and reaction mixture was vigorously stirred at reflux overnight. Reaction system was cooled down to room temperature and acetic acid was evaporated under reduced pressure. The organic residue was extracted with dichloromethane, organic phases were washed with water, dried over Na₂SO₄ and solvent was evaporated. The residue was recrystallized from acetone/ethanol mixture, solid material was filtered off and dried in *vacuo* at 60 °C affording 0.66 g (64% Yield) of final product with > 99% ee. ¹H NMR analysis was in agreement with known data.

### Example 9: Direct transformation of optically active 1-(3-ethoxy-4-methoxyphenyl)-2-(methylsulfonyl)ethanol (V) to apremilast

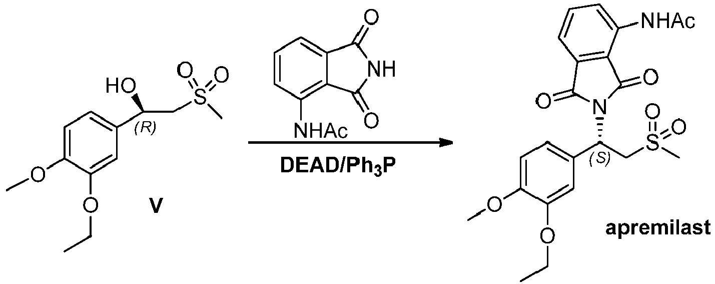

A 50 mL round bottom flask was charged with triphenylphosphine (1.75 mmol, 1.75 eq) and dichloromethane (3.4 mL). Afterwards 3-acetamidophthalimide (1 mmol, 1 eq) was added and the mixture was mixed at room temperature (rt). After 15 min optical active starting material 1-(3-ethoxy-4-methoxyphenyl)-2-(methylsulfonyl)ethanol (1 mmol, 1 eq; 98% optical purity) was added and the reaction mixture was cooled on ice. Later DEAD (40 % solution in toluene; 1.75 mmol, 1.75 eq) was added slowly and the mixture was mixed overnight at rt.
The following day a small amount of dichloromethane was added to the mixture, washed with brine and organic phase was dried over Na₂SO₄. The organic phase was evaporated in vacuo and 1.4 g of oily crude material was obtained. Crude material was diluted in ethylacetate and purified by column chromatography (SiO₂,EtOAc:n-heptane gradient elution) to afford yellowish solid (VIII) (22% yield) with 20% ee measured by chiral HPLC. Compound (VIII) was confirmed also with ¹H NMR spectroscopy.

### Comparative Example 1: Preparation of compound IV via β-borylation approach catalysed with copper(I) chloride in the presence of Ph₃P

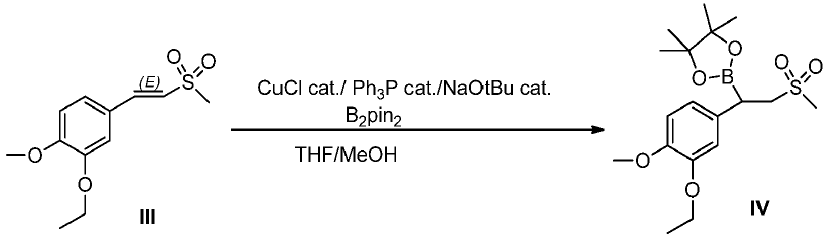

In a two-necked round bottom flask were placed catalyst CuCl (10 mol% according to starting material), NaO*t*Bu (2.92 mmol, 20 mol%) and Ph₃P (10 mol%) under the nitrogen. Afterward 2.5 mL of freshly degassed anhydrous THF was added and the reaction mixture was stirred (600 rpm) at ambient temperature for 30 min. Bis(pinacolato)diboron (B₂pin₂; 1.10 equiv.) was slowly added in two portions and after 30 min of stirring (green-yellow solution) unsaturated sulfone **(III)** (0.5 mmol) was slowly added. Such reaction mixture was stirred at ambient temperature for 30 min and afterwards protic additive MeOH (THF : MeOH = 2 : 1) was added and reaction system was maintained at ambient temperature and stirred for 20 hours (black solution). The reaction mixture was quenched with MeOH and immediately concentrated under the reduced pressure. Obtained crude material **(IV;** full conversion) was analysed with reverse-phase HPLC analysis (XBridge C18, CH₃CN/H₂O; 0.5mL/min; 40 °C) and determined with ¹H NMR.
¹H NMR (500 MHz, CDCl₃) δ 7.35 (m, 1H, ArH), 7.20 (m, 1H, ArH), 6.75 (m, 1H, ArH), 4.05 (q, 2H), 3.85 (s, 3H), 3.45 (m, 1H), 3.25 (m, 1H), 2.85 (m, 1H), 2.50 (s, 3H), 1.40 (t, 3H), 1.25 (s, 12H).

### Comparative Example 2: One-pot copper(I)-catalysed synthesis of 1-(3-ethoxy-4-methoxyphenyl)-2-(methylsulfonyl)ethanol (V) via β-borylation approach in the presence of Ph₃P

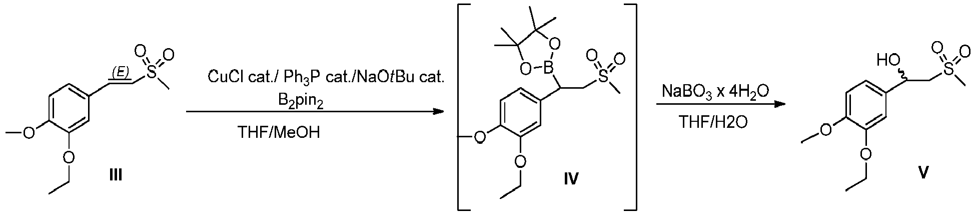

In a two-necked round bottom flask were placed catalyst CuCl (1.95 mmol, 193 mg, 10 mol%), NaO*t*Bu (2.92 mmol, 15 mol%) and Ph₃P (1.95 mmol; 10 mol%) under the nitrogen. Afterward 50 ml of freshly degassed anhydrous THF was added and the reaction mixture was stirred (600 rpm) at ambient temperature for 30 min. Bis(pinacolato)diboron (B₂pin₂; 1.10 equiv.) was slowly added in two portions and after 30 min of stirring (green-yellow solution) unsaturated sulfone **(III)** (19.5 mmol) was slowly added. Such reaction mixture was stirred at ambient temperature for 30 min and afterwards protic additive MeOH (25 ml; THF : MeOH = 2 : 1) was added and reaction system was maintained at ambient temperature and stirred for 20 hours (black solution). The reaction mixture was quenched with MeOH, filtered through Celite and concentrated under the reduced pressure. Obtained crude material **(IV)** was dissolved in THF : H₂O (1 : 1) and oxidizing agent (NaBO₃ x 4 H₂O; 3 equiv.) was added. Reaction mixture was stirred for 3 hours at ambient temperature. Afterwards solvent was evaporated, residue extracted with EtOAc, organic phases washed with brine and dried over Na₂SO₄. After evaporation of organic solvents the obtained crude product was purified with flash chromatography (EA : n-hexane; gradient elution) to obtain pure (**V**) (70% yield) as determined with ¹H NMR analysis.
¹H NMR (500 MHz, CDCl₃) δ 7.80-7.95 (m, 3H, ArH), 5.25 (m, 1H), 4.25 (q, 2H), 4.85 (s, 3H), 3.45 (m, 1 H), 3.20 (m, 1 H), 3.10 (s, 3H), 1.45 (t, 3H).

## Claims

1. A process for providing a compound of formula V, 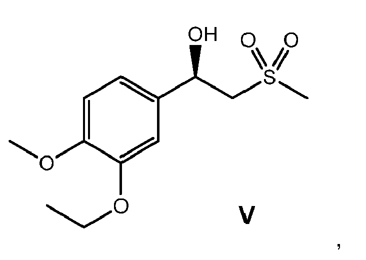 comprising
(a) providing a compound of formula III 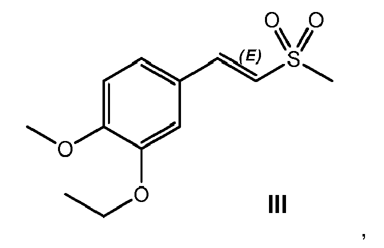
(b) reacting the compound of formula III with bis(pinacolato)diboron of formula 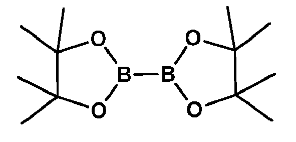 in the presence of a copper catalyst and a chiral phosphine ligand, to obtain a compound of formula IV 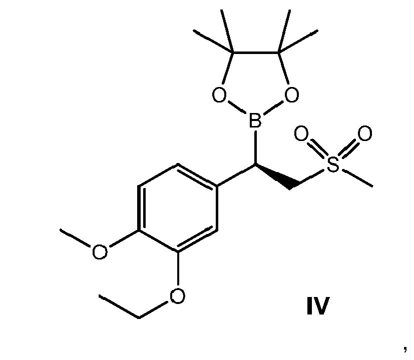 and
(c) oxidizing the compound of formula IV to obtain the compound of formula V.

2. The process according to claim 1, wherein the compound of formula IV is not isolated.

3. The process according to claim 1, wherein the copper catalyst is selected from the group consisting of CuCl, Cu(CH₃CN)₄BF₄, Cu(OAc)₂, and basic CuCO₃.

4. The process according to any of claims 1 to 3, wherein the chiral phosphine ligand is selected from the group consisting of P-P ligands BINAP, PPhos, XylPPhos, ferrocenyl type P-P ligands of Josiphos and Walphos type, and phosphine-oxazoline ferrocenyl type ligands FerroPhox.

5. The process according to claim 4, wherein the chiral phosphine ligand is a phosphine-oxazoline ferrocenyl type ligand FerroPhox.

6. The process according to claim 5, wherein the FerroPhox ligand has one of the following structures

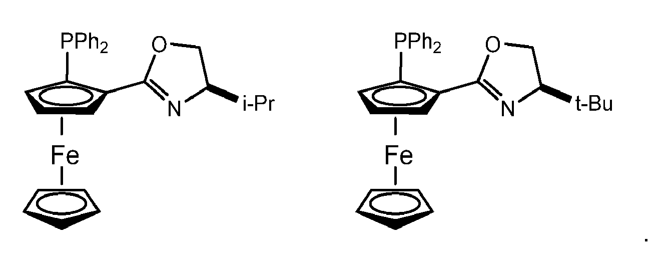

7. The process according to any of claims 1 to 6, wherein the oxidizing agent is selected from the group consisting of H₂O₂/NaOH, potassium peroxymonosulfate, NaOCl, and NaBO₃ x ₄H₂O.

8. The process according to claim 1, wherein the compound of formula III is obtained by providing the compound of formula I 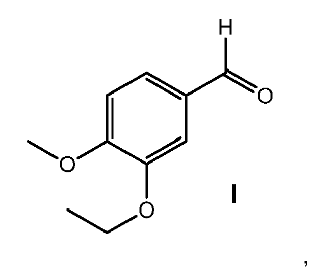 and
converting the compound of formula I into the compound of formula III.

9. The process according to claim 8, wherein the compound of formula I is first converted to the compound of formula II

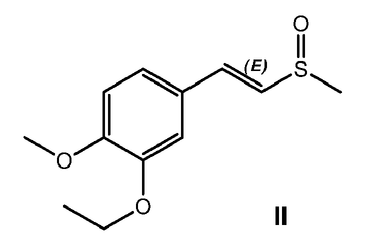

10. A process for preparing apremilast 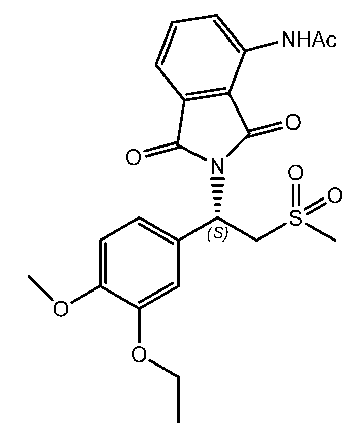 or a pharmaceutically acceptable salt or solvate thereof, comprising:
(aa) carrying out the process according to any one of the preceding claims, and
(bb) subsequently carrying out further synthetic step(s) to obtain apremilast or a pharmaceutically acceptable salt or solvate thereof.

11. The process according to claim 10, comprising
(i)' converting the compound of formula V 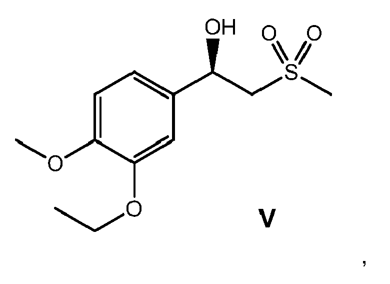 obtained by the process according to claim 1, into the compound of formula VI 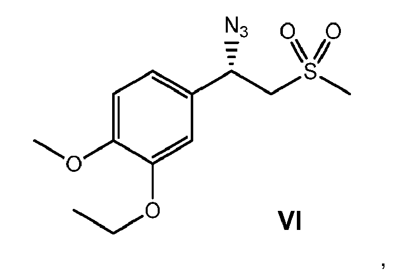
(ii)' reducing the compound of formula VI to obtain the compound of formula VII 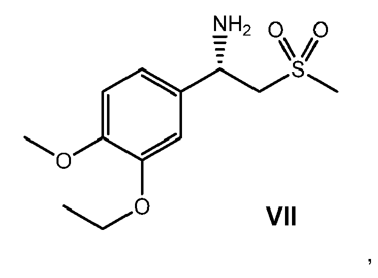 and
(iii)' reacting the compound of formula VII with 3-acetamidophthalic anhydride of formula 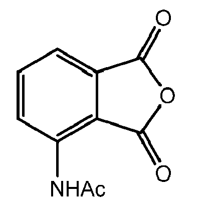 to obtain apremilast or a pharmaceutically acceptable salt or a solvate thereof.

12. The process according to claim 10, comprising
reacting the compound of formula V 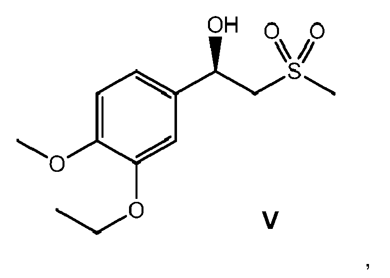 obtained by the process according to claim 1, with 3-acetamidophthalimide of formula 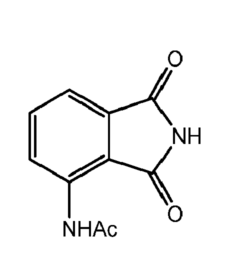 in the presence of a reagent selected from the group consisting of DEAD, DIAD and DCAD, in combination with Ph₃P and Bu₃P, to obtain apremilast or a pharmaceutically acceptable salt or a solvate thereof.

13. A compound of formula IV

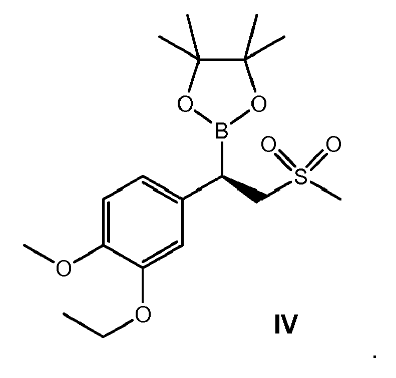

14. Use of the compound as defined in claim 13 in the preparation of apremilast or a pharmaceutically acceptable salt or a solvate thereof.

15. A process for the preparation of a pharmaceutical composition comprising apremilast or a pharmaceutically acceptable salt or a solvate thereof, comprising the steps of:
x) preparing apremilast or a pharmaceutically acceptable salt or a solvate thereof by carrying out a process according to claim 10, and
y) mixing said obtained apremilast or a pharmaceutically acceptable salt or a solvate thereof with a pharmaceutically acceptable carrier and/or excipient.
